# EUROPEAN PATENT APPLICATION

(11) **EP 3 961 639 A1**
(43) Date of publication of application: **02.03.2022**
(21) Application number: 20794656.7
(22) Date of filing: 18.04.2020
(51) Int. Cl.: G16H 10/60, G06F 16/904, G06F 3/0481, G06F 13/00, G06F 40/106

(54) **DOCUMENT DISPLAY SYSTEM**

(30) Priority: 20.04.2019 JP 2019080571
(71) Applicant: Iryou Jyouhou Gijyutu Kenkyusho Corporation, Yame-gun, Fukuoka 834-0102 (JP)
(72) Inventor: Himeno Shinkichi, Yame-gun Fukuoka 834-0102 (JP)
(74) Representative: Gulde & Partner
(86) International application number: PCT/JP2020/016976
(87) International publication number: WO 2020/218221

(57) **Abstract**

[Problem] To provide a document display system that assists a user in making an appropriate determination by efficiently displaying information required by the user at one time point among a variety and large amount of documentation or data on a screen easily settable by the user so that the user can easily grasp the situation and whose operation method is the same among devices from smartphones to PCs having large monitor screens and can be easily learned.

[Solving Means] The document display system includes (i) a multi-category document storage means that is storing a plurality of document categories and documents for each of the document categories, (ii) a unit display means comprising a unit display management means configured to manage independent unit display areas and a display portion related to multi-category document storage means displayed in the unit display areas, and (iii) a single page display means comprising a single page display management means configured to manage single page display areas in each of which the plurality of unit display means are arranged in one direction and that are each displayed as a single page and arrangement of the unit display means.

## Description

### TECHNICAL FIELD

The present invention relates to a document display system that displays a variety and large amount of documentation or data, such as electronic medical records, on the screen such that a user can easily use such documentation or data, as well as assists the user in efficiently making a decision or creating a new document.

### BACKGROUND ART

A variety of documentation or data, such as observation records of the conditions of patients, data on blood tests and imaging, the determination of diagnosis or treatment plans based on these, and execution instructions or the like of subsequent tests or treatment based on these, is created at medical sites and the like. These documents or the like are created by workers belonging to many job categories, such as doctor, nurse, care worker, rehabilitation staff, and medical affairs division staff. These workers have to grasp a treatment plan by reading not only records in their job category but also records in other job categories, to draft an action plan in their job category, and to create a document or record. Corporations and governments have similar problems in managing a one-case document relating to a complicated problem.

While documents were initially displayed on the monitor screens of desktop personal computers (PCs), cases in which it is necessary to reference or create a document outside a medical facility have been increased with the development of home medical care and the like. It has become indispensable to support mobile devices, such as smartphones and tablets.

Background art literature relating to the present application includes the following.

### Citation List

### Patent Literature

Patent Literature 1: Japanese Unexamined Patent Application Publication No. 2017 -220221
Patent Literature 2: Japanese Unexamined Patent Application Publication (Translation of PCT Application) No. 2016-535907
Patent Literature 3: Japanese Patent No. 4357829

### SUMMARY OF INVENTION

### Technical Problem

When the treatment of a patient, or a case is prolonged, a variety and large amount of documentation is generated. For this reason, when a worker in a certain job category attempts to obtain necessary information at a certain time point, the worker has to search for the necessary information, which is scattered in various parts of the large pile of documents. Such information collection work is hard, and the long working hours of medical and care staff members and fatigue associated with the long working hours have been problems. Of course, it has been a more difficult problem to share information among multiple job categories. For this reason, failures to search for such information have necessarily occurred, leading to accidents.

Use of a mobile device, such as a smartphone or tablet, allows a staff member to reference or create a document outside a medical institution. However, the screens of mobile devices and PCs have quite different sizes. For example, the difference in width between the work areas of the smallest smartphone and a large-screen PC is ten times or more. For this reason, the screen display method and the operation method vary between mobile devices and PCs, and staff members have to take much effort to learn the respective operation methods of the mobile devices and PCs, which differ from each other.

The present invention has been made to solve the above problem with the background art, and an object thereof is to provide a document display system that assists a user in one job category in making an appropriate determination by efficiently displaying information required by the user at one time point among a variety and large amount of documentation or data on a screen easily settable by the user so that the user can easily grasp the situation and whose operation method is the same among devices from smartphones to PCs having large monitor screens and can be easily learned.

### Solution to Problem

As a means for accomplishing the above object, a document display system includes (i) a multi-category document storage means that is storing multiple document categories and documents for each of the document categories, (ii) a unit display means including a unit display management means configured to manage independent unit display areas and a display portion related to multi-category document storage means displayed in the unit display areas, and (iii) a single page display means including a single page display management means configured to manage single page display areas in each of which the multiple unit display means are arranged in one direction and that are each displayed as a single page and arrangement of the unit display means.

According to the document display system of claim 2, in the document display system of claim 1, the unit display management means is configured such that a user is able to specify, as content displayed in the unit display area, not only the display portion related to multi-category document storage means but also a display portion of another multi-category document storage means, web content specified by a Uniform Resource Locator (URL) or the like, an email or a social networking service (SNS) available through a Web service, and content on a local storage medium.

According to the document display system of claim 3, in the document display system of claim 1 or 2, the multi-category document storage means is configured such that a user is able to specify, as the display portion thereof, one of (i) all of a specified document, (ii) a specified portion of a specified document, (iii) a list of documents recorded as a specified category, and (iv) documents created in a specified period of time and included in a specified document category.

According to the document display system of claim 4, in the document display system of any one of claims 1 to 3, the unit display means is configured such that a user is able to form a part or all of displayed content into an object and to copy the object to another unit display means by drag-and-drop the object.

According to the document display system of claim 5, in the document display system of any one of claims 1 to 4, the unit display management means is configured such that a user is able to make a selection as to whether a change made to content displayed in the unit display area is (i) automatically reflected on display or (ii) reflected on display only when an explicit user trigger is made.

According to the document display system of claim 6, in the document display system of any one of claims 1 to 5, the single page display management means includes an in-single-page unit display arrangement management means configured to manage the arrangement of the unit display means.

According to the document display system of claim 7, in the document display system of any one of claims 1 to 5, the single page display management means includes an in-single-page unit display size management means configured to manage display sizes of the individual unit display means.

According to the document display system of claim 8, the document display system of any one of claims 1 to 7 includes a simultaneous parallel single page screen display means including a simultaneous parallel single page screen management means configured to manage a simultaneous parallel single page screen display area in which the multiple single page display means are displayed simultaneously in parallel and the single page display means displayed simultaneously in parallel.

According to the document display system of claim 9, in the document display system of claim 8, the simultaneous parallel single page screen management means includes a single page display area size specification means configured such that a user is able to specify sizes of the individual single page display areas.

According to the document display system of claim 10, in the document display system of claim 8 or 9, the simultaneous parallel single page screen management means includes a simultaneous parallel single page screen selection means configured such that a user is able to select an appropriate simultaneous parallel single page screen from multiple registered single page simultaneous parallel screens.

### Advantageous Effects of Invention

The document display system of claim 1 includes the multi-category document storage means and thus is storing documents for each of the document categories.

The document display system of claim 1 also includes the unit display management means and thus manages the independent unit display areas and the display portion related to multi-category document storage means displayed on the unit display areas.

The document display system of claim 1 also includes the single page display management means and thus manages the single page display areas in each of which the multiple unit display means are arranged in one direction and that are each displayed as a single page and the arrangement of the unit display means.

In the document display system of claim 2, the unit display management means is configured such that a user is able to specify, as content displayed in the unit display area, not only the display portion related to multi-category document storage means but also a display portion of another multi-category document storage means, web content specified by a Uniform Resource Locator (URL) or the like, an email or a social networking service (SNS) available through a Web service, and content on a local storage medium.

In the document display system of claim 3, the multi-category document storage means is configured such that a user is able to specify, as the display portion thereof, one of (i) all of a specified document, (ii) a specified portion of a specified document, (iii) a list of documents recorded as a specified category, and (iv) documents created in a specified period of time and included in a specified document category.

In the document display system of claim 4, the unit display means is configured such that a user is able to form a part or all of displayed content into an object and to copy the object to another unit display means by drag-and-drop the object.

In the document display system of claim 5, the unit display management means is configured such that a user is able to make a selection as to whether a change made to content displayed in the unit display area is (i) automatically reflected on display or (ii) reflected on display only when an explicit user trigger is made.

In the document display system of claim 6, the single page display management means includes the in-single-page unit display arrangement management means and thus manages the arrangement of the unit display means.

The document display system of claim 7 includes the in-single-page unit display size management means and thus manages the display sizes of the unit display means.

The document display system of claim 8 includes the simultaneous parallel single page screen display area and thus the single page display means are displayed simultaneously in parallel.

The document display system of claim 8 also includes the simultaneous parallel single page screen management means and thus manages the single page display means displayed simultaneously in parallel.

The document display system of claim 8 includes the single page display area size specification means and thus the user is able to specify the sizes of the individual single page display areas.

The document display system of claim 10 includes the simultaneous parallel single page screen selection means and thus the user is able to select an appropriate simultaneous parallel single page screen from the multiple registered single page simultaneous parallel screens.

### Brief Description of the Drawings

FIG. 1 is a diagram showing a single page display means and unit display means of the present invention.
FIG. 2 is a diagram showing the display portion related to multi-category document storage means.
FIG. 3 is a diagram showing formation of content related to a unit display means into an object.
FIG. 4 is a diagram showing a unit display management means.
FIG. 5 is a diagram showing a single page display management means.
FIG. 6 is a diagram showing a simultaneous parallel single page screen display means.
FIG. 7 is a diagram showing a simultaneous parallel single page screen management means.
FIG. 8 is a diagram showing a deformed unit display area.

### DESCRIPTION OF EMBODIMENTS

The present application is a system that runs on a computer.

This computer includes an input device (mouse, keyboard, etc.), an output device (monitor, printer, etc.), a storage device (memory, hard disk), a computing device (CPU), a controller (CPU), and the like, as well as includes a program for performing the means (functions) of the present application.

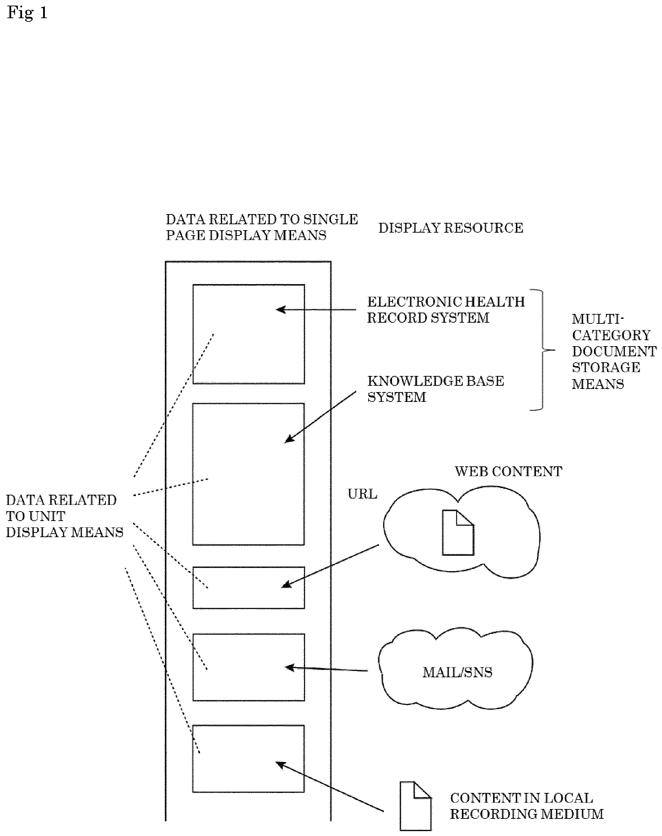

FIG. 1 is a diagram showing a single page display means and unit display means of the present invention.

Multiple rectangular display areas (unit displays) are arranged in one direction (here, the longitudinal direction; may be the lateral direction) and form a single page display.

The respective unit display means are associated with specified portions of an electronic medical record system, knowledge base system, or the like, which is a multi-category document storage means. An addition or change made to a source document is reflected on a corresponding unit display.

While the document display system of the present invention mainly displays documents in the multi-category document storage means, it is also able to display web content documents specified by URLs, common message transmission/reception tools, such as emails and SNS, and local content in the hard disk of a PC or in a USB, CD, Blu-ray Disc, or the like to assist a user in making a decision or creating a document.

Thus, multiple tools or systems can be integrated into the document display system of the present invention, and the user can avoid complications associated with displaying multiple tools or systems separately and switching therebetween.

If multiple systems or tools are allowed to be logged in by authentication through one-time input of ID and password called single sign-on, the utility of the document display system of the present invention is further increased.

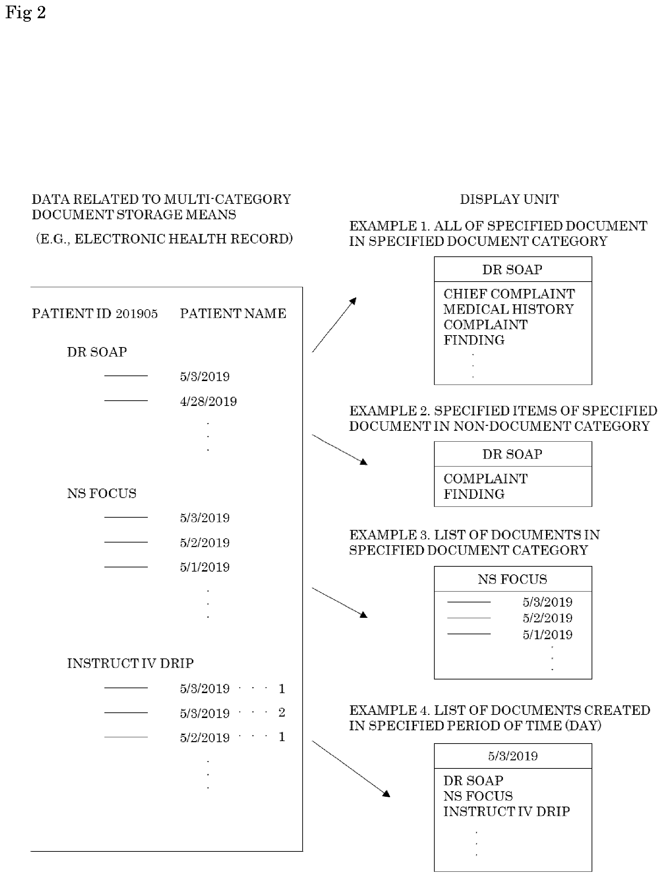

FIG. 2 is a diagram showing a specified display portion of an electronic medical record, which is related to multi-category document storage means.

Example 1 shows all of a specified document (typically, the latest document; the date and time or the like may be specified) in a specified document category (here, Dr. SOAP). Note that multiple documents, for example, three documents until the latest one, may be specified.

Example 2 shows one portion extracted from the above specified document as a specified item.

If the document is an XML or JSON document, a tag such as < > or : is used to specify an item. The heading or the like of the document data may be used. As in Example 1, multiple documents, for example, three documents until the latest one, may be specified.

Example 3 shows a list of documents included in the specified document category.

If not only the date of each document but also characters at the top of the document are displayed together or the contents of the document are displayed on a pop-up screen or the like by mouseover, the user is able to estimate the contents of each document and then to easily select a document.

The contents of a selected document can be displayed and edited on a differentdata.

Example 4 lists documents created in a specified period of time (here, one day on May 3) in parallel regardless of the document category.

The contents of a selected document can be displayed and edited on a different unit display means.

The document display system is useful to show the flow of documents created with respect to one case in a calendar form. To prevent displayed document categories from being increased in number and complicated, the number of displayed document categories may be restricted. Documents may be displayed on different unit display means on a date or category basis. The unit display means may be managed as separate data for each date or category. One unit display may be divided into dates and document category groups in a grid.

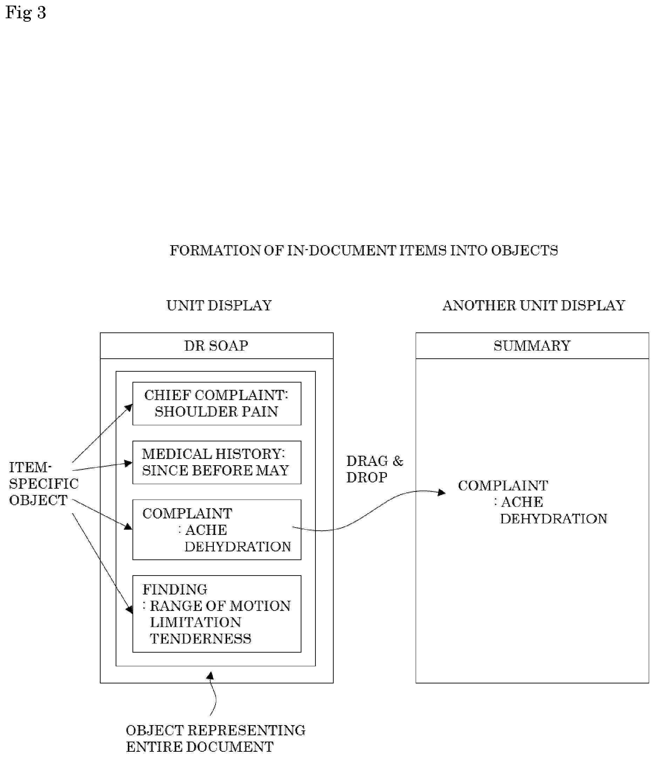

FIG. 3 is a diagram showing formation of content related to a unit display means into objects.

While a large amount of transfer work occurs when creating a document for each case, the contents of the source document are efficiently transferred by forming the items of the source document into objects and drag-and-drop the objects onto the destination document.

If the source document is an XML or JSON document, tags such as < > or : are used to specify the items. The heading or the like of the document data may be used.

Also, by previously forming the description items of the destination document into objects and drag-and-drop the above objects into the description item objects of the destination document, the efficiency is further increased.

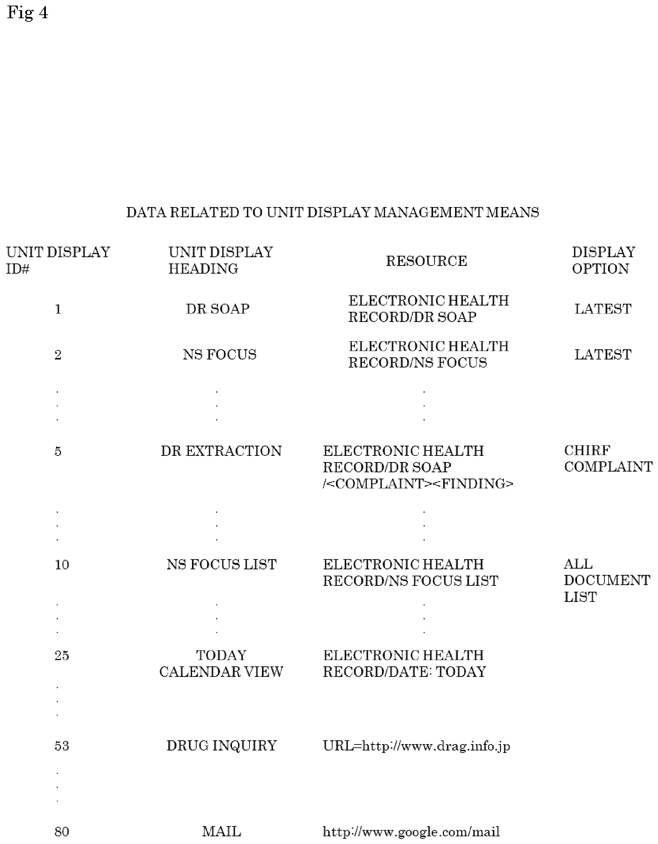

FIG. 4 is a diagram showing a unit display management means.

By allowing each user to define a unit display displayed in a single page, the user is able to create information display layout according to the user's preference without having to make a request to the system department (consumer defined user interface).

As shown in FIG. 4, with respect to frequently used unit displays, the display resources and display options thereof may be previously stored and managed using unit display ID Nos. and unit display headings. Thus, the user is able to reuse a required unit display by only selecting the style of the unit display.

The user may define the style of a unit display according to the user's preference and register and reuse the unit display using the style. Also, it is useful to make the style of the unit display public to other users so that the other users can also use it.

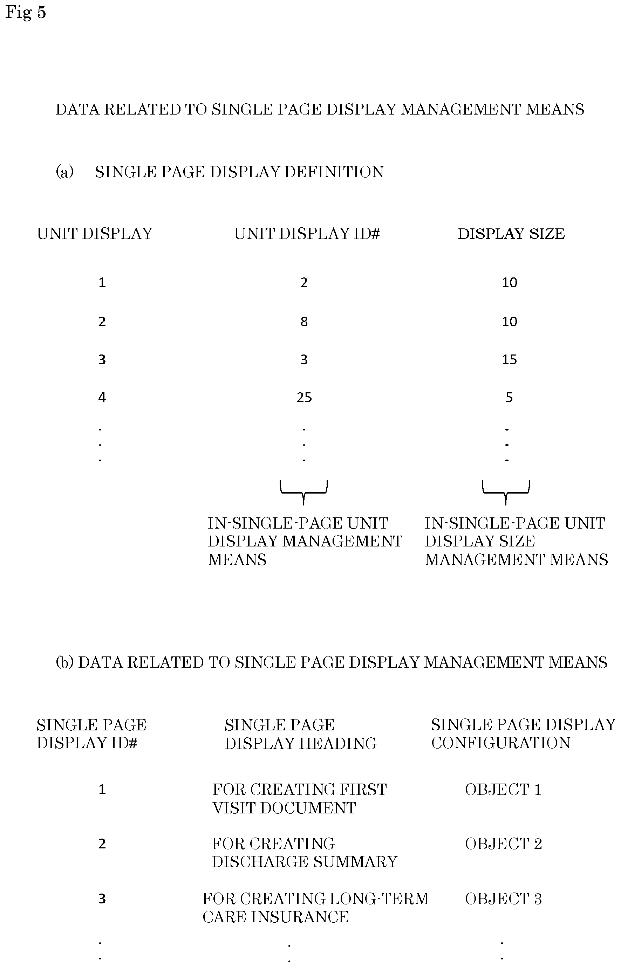

FIG. 5 is a diagram showing a single page display management means.

(a) shows the definition of one single page display.

More specifically, (a) shows the serial numbers of unit displays arranged in one direction in this single page display means and resources displayed on the unit displays using the unit display ID Nos. of the unit displays(shown in FIG. 4) (in-single-page unit display management means).

(a) also shows the specified numbers of lines of the unit displays (in-single-page unit display size management means).

In (b), pieces of single page display management information as shown in (a) are organized and formed into objects by XML, JSON, or the like and managed using IDs# and headings.

By storing display patterns such as unit display arrangements in each single page display means and managing the single page display means using single page display ID Nos. and single page display headings as shown in (b), the user is able to reuse a required single page display means by only selecting the style of the single page display means.

The user may define the style of a single page display means according to the user's preference and register and reuse the single page display means using the style.

Also, it is useful to make the style of the single page display means public to other users so that the other users can also use it.

The user may use any of the unit display management means of FIG. 4 or the single page display management means of (a) to make a selection as to whether a change made to a display resource is automatically reflected on a corresponding unit display means, or reflected thereon on the basis of an explicit user trigger since it is troublesomeness that a change is made to the display during work.

While FIG. 4 shows an example in which all the unit displays in the document display system of the present invention are managed by the unit display management means, a unit display management means may be set for each of single page display management means as shown in FIG. 5.

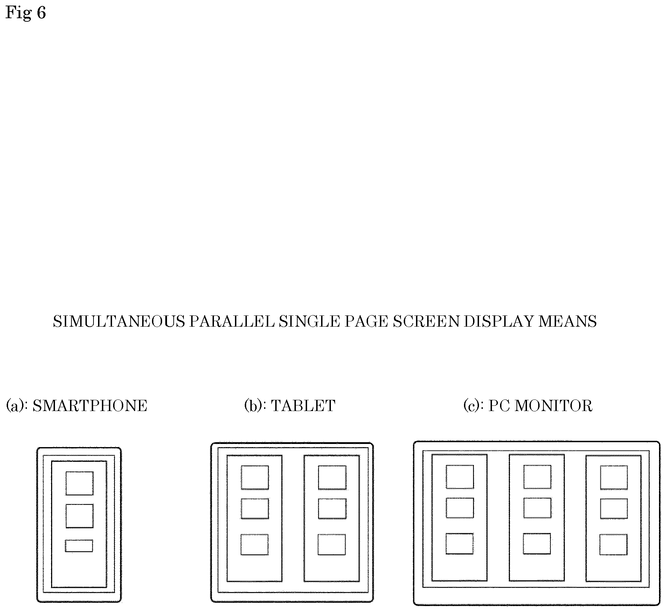

FIG. 6 is a diagram showing simultaneous parallel single page screen display means.

(a) shows an example of the display of a smartphone. Since the display screen of the smartphone is small, it is appropriate to display one columnar single page display.

Of course, two columnar single page displays may be set simultaneously in parallel. In this case, only one columnar single page display is displayed, and, when necessary, the other single page display is displayed by shifting the screen.

(b) shows an example of the display of a tablet. In this example, two columnar single page displays are displayed. Of course, a larger number of columnar single page displays may be set. In this case, when necessary, a single page display hidden from the screen is displayed by shifting the screen.

As an example usage of this display, object items on the first column may be transferred to a new document shown as the second column, for example, by drag-and-drop them as shown in FIG. 3.

(c) shows an example of the display of the monitor screen of a large PC. In this example, three or more columns are displayed simultaneously in parallel. Of course, a larger number of single page displays may be set. In this case, when necessary, a single page display hidden from the screen is displayed by shifting the screen.

A variety of information can be displayed in a developed form, and a new document can be created.

As seen above, single page displays are displayed simultaneously in parallel regardless of the size of the screen as necessary. Thus, the user is able to use devices including smartphones, which have small screens, and PCs, which have large screens, using the same operation method and to easily learn the operation method.

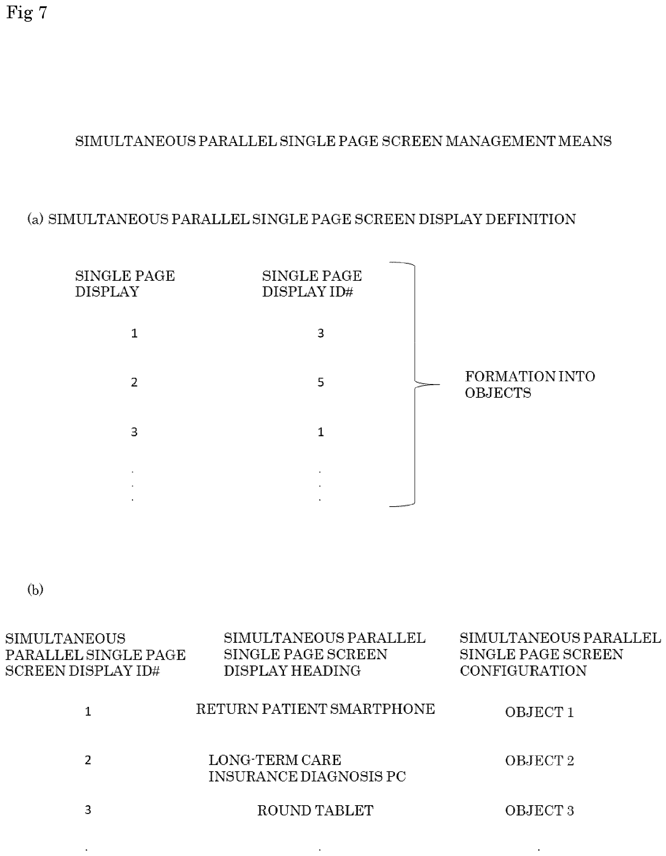

FIG. 7 is a diagram showing a simultaneous parallel single page screen management means.

(a) shows the definition of one simultaneous parallel single page screen.

In (a), single page displays arranged on the simultaneous parallel single page screen are shown using the serial numbers and single page display ID Nos. thereof (in-simultaneous-parallel-single-page-screen single page display management means).

In (b), pieces of information on the definitions of the simultaneous parallel single page screens shown in (a) are organized and formed into objects by XML, JSON, or the like, and the objects are managed using simultaneous parallel single page screen ID Nos. and headings.

By storing display patterns such as single page display arrangements in each simultaneous parallel single page screen and managing them using simultaneous parallel single page screen display ID Nos. and simultaneous parallel single page screen display headings as shown in FIG. 7, the user is able to reuse a required simultaneous parallel single page screen display by only selecting the style of the simultaneous parallel single page screen display.

The user may define the style of a simultaneous parallel single page screen display according to the user's preference and register and reuse the simultaneous parallel single page screen display using the style.

Also, it is useful to make the style of the simultaneous parallel single page screen display public to other users so that the other users can also use it.

While an embodiment of the present invention has been described, the specific configuration of the present invention is not limited to the embodiment. Design changes and the like in the embodiment are included in the present invention without departing from the spirit and scope of the invention.

For example, while the electronic medical record system, knowledge base system, and the like have been used as examples of the multi-category document storage means, the multi-category document storage means is not limited thereto. The present invention can also display a document (common case records, etc.) recorded for each case including multiple document categories.

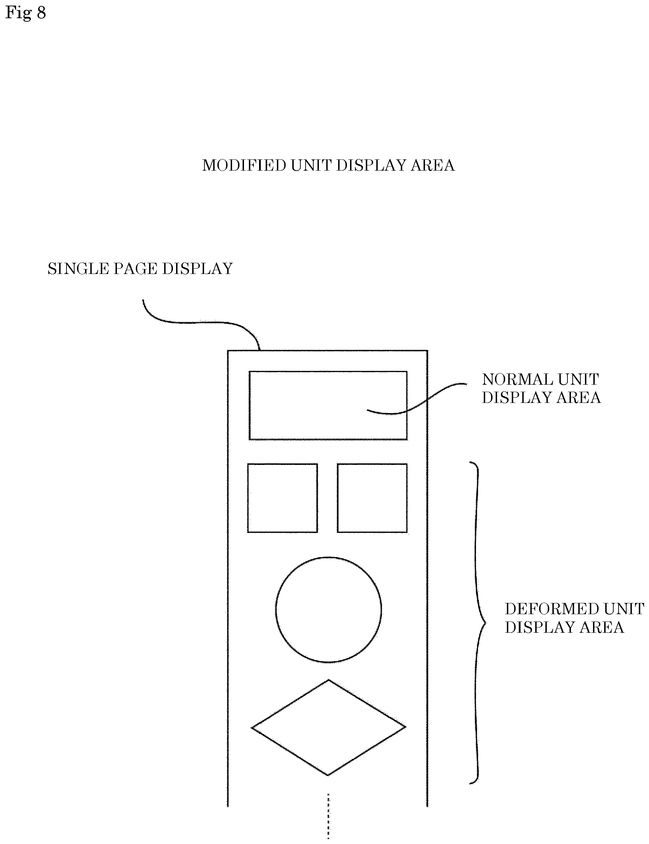

While each unit display area has a rectangular shape having the width of a corresponding single page display in the above embodiment, a deformed unit display area as shown in FIG. 8 is also included in the present invention.

## Claims

1. A document display system comprising:
(i) a multi-category document storage means that is storing a plurality of document categories and documents for each of the document categories;
(ii) a unit display means comprising a unit display management means configured to manage independent unit display areas and a display portion related to multi-category document storage means displayed in the unit display areas; and
(iii) a single page display means comprising a single page display management means configured to manage single page display areas in each of which the plurality of unit display means are arranged in one direction and that are each displayed as a single page and arrangement of the unit display means.

2. The document display system of claim 1, wherein
the unit display management means is configured such that a user is able to specify, as content displayed in the unit display area, not only the display portion related to multi-category document storage means but also a display portion related to another multi-category document storage means, web content specified by a Uniform Resource Locator (URL) or the like, an email or a social networking service (SNS) available through a Web service, and content on a local storage medium.

3. The document display system of claim 1 or 2, wherein
the multi-category document storage means is configured such that a user is able to specify, as the display portion thereof, one of (i) all of a specified document, (ii) a specified portion of a specified document, (iii) a list of documents recorded as a specified category, and (iv) documents created in a specified period of time and included in a specified document category.

4. The document display system of any one of claims 1 to 3, wherein the unit display means is configured such that a user is able to form a part or all of displayed content into an object and to copy the object to another unit display means by drag-and-drop the object.

5. The document display system of any one of claims 1 to 4, wherein the unit display management means is configured such that a user is able to make a selection as to whether a change made to content displayed in the unit display area is (i) automatically reflected on display or (ii) reflected on display only when an explicit user trigger is made.

6. The document display system of any one of claims 1 to 5, wherein the single page display management means comprises an in-single-page unit display arrangement management means configured to manage the arrangement of the unit display means.

7. The document display system of any one of claims 1 to 5, wherein the single page display management means comprises an in-single-page unit display size management means configured to manage display sizes of the individual unit display means.

8. The document display system of any one of claims 1 to 7, comprising a simultaneous parallel single page screen display means comprising a simultaneous parallel single page screen management means configured to manage a simultaneous parallel single page screen display area in which the plurality of single page display means are displayed simultaneously in parallel and the single page display means displayed simultaneously in parallel.

9. The document display system of claim 8, wherein the simultaneous parallel single page screen management means comprises a single page display area size specification means configured such that a user is able to specify sizes of the individual single page display areas.

10. The document display system of claim 8 or 9, wherein the simultaneous parallel single page screen management means comprises a simultaneous parallel single page screen selection means configured such that a user is able to select an appropriate simultaneous parallel single page screen from a plurality of registered single page simultaneous parallel screens.
